(19) Europäisches Patentamt
European Patent Office
Office européen des brevets

(11) **EP 3 375 357 B1**

(12) **EUROPEAN PATENT SPECIFICATION**

(45) Date of publication and mention
of the grant of the patent:
**25.01.2023 Bulletin 2023/04**

(21) Application number: **15909532.2**

(22) Date of filing: **03.12.2015**

(51) International Patent Classification (IPC):
*A61B 5/02* (2006.01)      *A61B 5/024* (2006.01)
*A61B 5/11* (2006.01)      *A61B 5/00* (2006.01)

(52) Cooperative Patent Classification (CPC):
**A61B 5/1118; A61B 5/02438; A61B 5/7289**

(86) International application number:
**PCT/CN2015/096362**

(87) International publication number:
**WO 2017/092018 (08.06.2017 Gazette 2017/23)**

(54) **BIOLOGICAL SIGNAL ACQUISITION METHOD, DEVICE, ELECTRONIC EQUIPMENT AND SYSTEM**

VERFAHREN ZUR ERFASSUNG BIOLOGISCHER SIGNALE, VORRICHTUNG, ELEKTRONISCHE AUSRÜSTUNG UND SYSTEM

PROCÉDÉ, DISPOSITIF, ÉQUIPEMENT ÉLECTRONIQUE ET SYSTÈME D'ACQUISITION DE SIGNAUX BIOLOGIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**19.09.2018 Bulletin 2018/38**

(73) Proprietor: **Huawei Technologies Co., Ltd.
Longgang District
Shenzhen, Guangdong 518129 (CN)**

(72) Inventors:
• **XU, Peida
Shenzhen
Guangdong 518129 (CN)**

• **CHEN, Wenjuan
Shenzhen
Guangdong 518129 (CN)**

(74) Representative: **Körber, Martin Hans
Mitscherlich PartmbB
Patent- und Rechtsanwälte
Sonnenstrasse 33
80331 München (DE)**

(56) References cited:
**EP-A1- 2 229 880       WO-A1-2015/058923
WO-A1-2015/131065     WO-A1-2016/010652
CN-A- 103 699 795       CN-A- 103 767 710
CN-U- 202 207 139       US-B2- 8 622 922**

## Description

### TECHNICAL FIELD

Embodiments of the present invention relate to the field of communications technologies, and more specifically, to a biological signal collection method, apparatus, and system, and an electronic device.

### BACKGROUND

With emergence of problems such as population aging, subhealth, and environmental pollution, people's requirement and concern for health are increasingly high. The internet, intelligent terminals, wearable devices, and medical informatization rapidly develop, so that mobile health becomes an important development direction, and increasingly more attention is paid on development and promotion of mobile health devices in national and foreign markets. For the mobile health, biological signal collection of a user is an extremely important aspect, and is a start point of a subsequent whole information processing process.

Biological signal collection duration is a key indicator. Extremely long collection duration affects user experience. Extremely short collection duration may cause an insufficient quantity of obtained signal values, and consequently a relatively large error is caused, and precision is hard to ensure. Currently, all wearable devices in a mobile health market use fixed duration when collecting a biological signal. The fixed duration is mostly determined according to a market requirement of a product or an idea of a technical expert, and has relatively strong subjectivity. Therefore, it is necessary to properly determine biological signal collection duration, so as to ensure both user experience and device precision.

Document WO 2015/058923 A1 discloses a device and a method for estimating the energy expenditure of a person with an increased accuracy, in which a movement sensor is adapted to detect a physical activity of a user and a control unit for switching on/off according to the detection of physical activity of the user.

Document WO 2015/131065 A1 discloses a method for improving the quality of physiological assessments using physical activity and biometric parameters.

Document WO 2016/010652 A1 is prior art relevant under Art. 54(3) EPC only and discloses a mobile device for energy efficient heart rate data collection.

### SUMMARY

This application provides a biological signal collection method, apparatus, and system, and an electronic device, so as to properly control biological signal collection duration, and improve biological signal measurement precision.

The present invention is defined by a method for biological signal collection according to independent claim 1 and 3 and a system for biological signal collection according to independent claim 6 and 8. Advantageous features are defined in the dependent claims.

According to a first aspect of the disclosure, an embodiment of this application provides a biological signal collection method, and the method includes: obtaining output data of at least one motion sensor; controlling collection duration of at least one biological signal of a user according to at least the output data of the at least one motion sensor; and collecting the at least one biological signal of the user in the collection duration of the at least one biological signal. In a motion state, the user is easily affected by electromyogram noise and a motion artifact. In this case, biological signal quality is worse than quality in a motionless state. Biological signal collection duration of the user is controlled based on motion status information that is of the user and that is collected by the motion sensor, so as to flexibly control the biological signal collection duration, and further improve biological signal measurement precision. The motion sensor may include any one of an accelerometer, a gyroscope, a pressure sensor, a microphone, a magnetometer, or an altimeter.

According to the first aspect, in a first possible implementation of the biological signal collection method, at least one of an activity type or an activity intensity of the user may be identified according to at least the output data of the at least one motion sensor, first duration that matches the at least one of the activity type or the activity intensity of the user is obtained, and the at least one biological signal of the user is collected according to the first duration. The activity type may include various examples, such as running, walking, cycling, swimming, climbing, standing, sitting, and sleeping. Generally, any case that describes an action and/or movement of the user may be referred to as an "activity". For different activity types and different activity intensities, a same biological signal differently changes and is differently affected by the electromyogram noise and the motion artifact. Proper collection duration is selected according to a current activity type and/or a current activity intensity of the user, so as to further improve biological signal measurement precision.

According to the first aspect, in a second possible implementation of the biological signal collection method, the at least one biological signal is periodic, for example, an electrocardiogram (ECG) signal, or a pulse wave (PPG) signal. Usually, for a periodic biological signal, measurement precision can be ensured only when an enough quantity of complete waveforms are collected. To obtain an enough quantity of complete waveforms, a quantity of feature reference points of a collected biological signal may be detected. When the quantity of feature reference points reaches a specified quantity, biological signal collection is stopped, so as to ensure measurement precision. Similar to the first possible implementation of the first aspect, at least one of an activity type or an activity intensity of the user may be identified according to at least the output data of the at least one motion sensor, a first value that matches the at least one of the activity type or the activity intensity of the user is obtained, a quantity of feature reference points of the at least one biological signal is detected, and collection of the at least one biological signal is stopped when the quantity of feature reference points is equal to the first value. Proper collection duration is selected according to a current activity type and/or a current activity intensity of the user, so as to further improve precision of measuring a periodic biological signal.

According to a second aspect, an embodiment of this application provides a biological signal collection apparatus. The collection apparatus has a function of implementing the method in any one of the first aspect or the implementations of the first aspect. The function may be implemented by using hardware, or may be implemented by executing corresponding software by hardware. The hardware or the software includes one or more modules corresponding to the function. In a possible design, the apparatus includes: an obtaining unit, configured to obtain output data of at least one motion sensor; a control unit, configured to control collection duration of at least one biological signal of a user according to at least the output data of the at least one motion sensor; and a collection unit, configured to collect the at least one biological signal of the user in the collection duration of the at least one biological signal.

According to a third aspect, an embodiment of this application provides an electronic device. The electronic device has a function of implementing the method in any one of the first aspect or the implementations of the first aspect. The function may be implemented by using hardware, or may be implemented by executing corresponding software by hardware. The hardware or the software includes one or more modules corresponding to the function.

In a possible design, the electronic device includes: at least one motion sensor, configured to monitor motion of a user; a memory, configured to store an instruction or data; a processor, coupled to the memory, where the processor is configured to implement the following functions in any one of the first aspect or the implementations of the first aspect: obtaining output data of the at least one motion sensor; controlling collection duration of at least one biological signal of the user according to at least the output data of the at least one motion sensor; and at least one biosensor, configured to collect the at least one biological signal of the user in the collection duration of the at least one biological signal.

According to a fourth aspect, an embodiment of this application provides a biological signal collection system. The system has a function of implementing the method in any one of the first aspect or the implementations of the first aspect. The system includes: at least one motion sensor, configured to monitor motion of a user; a memory, configured to store an instruction or data; a processor, coupled to the memory, where the processor is configured to implement the following functions in any one of the first aspect or the implementations of the first aspect: obtaining output data of the at least one motion sensor; controlling collection duration of at least one biological signal of the user according to at least the output data of the at least one motion sensor; and at least one biosensor, configured to collect the at least one biological signal of the user in the collection duration of the at least one biological signal.

According to the fourth aspect, in a first possible implementation of the biological signal collection system, the at least one motion sensor is coupled to the processor by using a wireless interface.

According to the fourth aspect, in a second possible implementation of the biological signal collection system, the at least one motion sensor is coupled to the processor by using a wired interface.

According to the fourth aspect or the first or the second implementation of the fourth aspect, in a third possible implementation of the biological signal collection system, the at least one biosensor is coupled to the processor by using a wireless interface.

According to the fourth aspect or the first or the second implementation of the fourth aspect, in a fourth possible implementation of the biological signal collection system, the at least one biosensor is coupled to the processor by using a wired interface.

According to any one of the fourth aspect or the implementations of the fourth aspect, in a fifth possible implementation of the biological signal collection system, the at least one motion sensor and the processor are disposed on a same device, or separately disposed on different devices.

According to any one of the fourth aspect or the implementations of the fourth aspect, in a sixth possible implementation of the biological signal collection system, the at least one biosensor and the processor are disposed on a same device, or separately disposed on different devices.

According to a fifth aspect, an embodiment of the present invention provides a computer storage medium, configured to store a computer software instruction used by the foregoing electronic device, and the computer storage medium includes a program designed for performing the method in any one of the first aspect or the implementations of the first aspect.

In comparison with the prior art, in solutions provided in the present invention, biological signal collection duration can be flexibly controlled, and biological signal measurement precision can be improved.

**BRIEF DESCRIPTION OF DRAWINGS**

To describe the technical solutions in the embodiments of the present invention more clearly, the following briefly describes the accompanying drawings required for describing the embodiments or the prior art. Apparently, the accompanying drawings in the following description show merely some embodiments of the present invention, and a person of ordinary skill in the art may still derive other drawings from these accompanying drawings without creative efforts.

FIG. 1 is a schematic structural diagram of an electronic device according to an embodiment of the present invention;

FIG. 2 is a flowchart of a biological signal collection method according to an embodiment of the present invention;

FIG. 3 is a flowchart of another biological signal collection method according to an embodiment of the present invention;

FIG. 4 is a flowchart of an activity type identification method according to an embodiment of the present invention;

FIG. 5 is a schematic diagram of an electrocardiogram signal;

FIG. 6 is a schematic diagram of a pulse wave signal;

FIG. 7 is a schematic diagram of a waveform of a whole electrocardiogram signal;

FIG. 8 is a schematic diagram of a pulse arrival time;

FIG. 9 is a flowchart of still another biological signal collection method according to an embodiment of the present invention;

FIG. 10 is a schematic structural diagram of a biological signal collection apparatus according to an embodiment of the present invention;

FIG. 11 is a schematic structural diagram of another electronic device according to an embodiment of the present invention;

FIG. 12 is a schematic structural diagram of a biological signal collection system according to an embodiment of the present invention; and

FIG. 13 is a schematic diagram of a specific application scenario of a biological signal collection system according to another embodiment of the present invention.

**DESCRIPTION OF EMBODIMENTS**

The following clearly and completely describes the technical solutions in the embodiments of the present invention with reference to the accompanying drawings in the embodiments of the present invention.

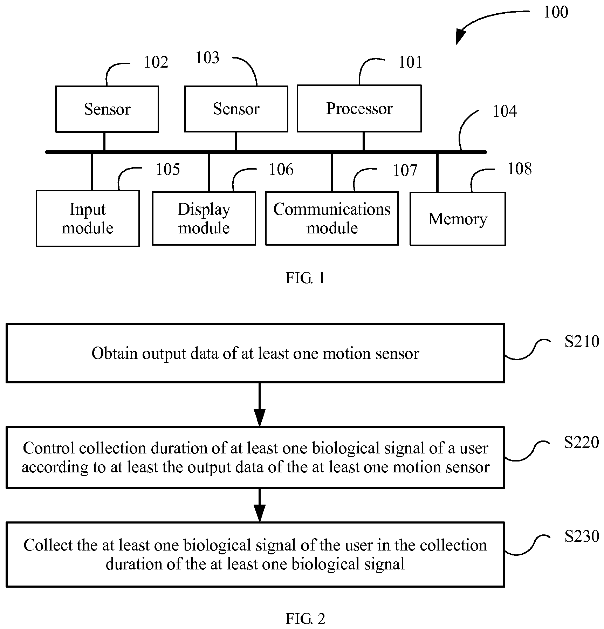

FIG. 1 is a module diagram of an electronic apparatus according to an embodiment of the present invention. Referring to FIG. 1, an electronic device 100 may include a processor 101, a bus 104, a memory 108, and a sensor 102.

The memory 108 may include one or more storage media, for example, include a hard disk drive, a solid-state drive, a flash memory, a persistent memory such as a read-only memory ("ROM"), a semi-persistent memory such as a random access memory ("RAM"), any other proper type of storage component, or any combination thereof. The memory 108 may be a built-in memory or an external memory. The built-in memory may include at least one of a volatile memory such as a dynamic random access memory (DRAM: dynamic RAM), a static random access memory (SRAM: static RAM), or a synchronous dynamic random access memory (SDRAM synchronous dynamic RAM), or a nonvolatile memory (nonvolatile Memory) such as a one time programmable read-only memory (OTPROM: one time programmable ROM), a programmable read-only memory (PROM: programmable ROM), an erasable programmable read only memory (EPROM: erasable programmable ROM), an electrically erasable programmable read only memory (EEPROM: electrically erasable programmable ROM), a mask read-only memory (mask ROM), a flash read-only memory (flash ROM), a NAND flash memory (NAND flash memory), or a NOR flash memory (NOR flash memory). In this case, the built-in memory may also be in a form of a solid-state drive (SSD: Solid-State Drive). The external memory may include at least one of compact flash (CF: compact flash), a secure digital (secure digital) card, a micro secure digital (microSD: micro secure digital) card, a mini secure digital (miniSD: mini secure digital) card, an extreme digital (xD: extreme digital) card, or a memory stick (memory stick).

Optionally, the electronic device 100 includes more than one sensor (for example, the sensor 102 and a sensor 103 in FIG. 1). For example, the sensor 102 is a motion sensor, and is configured to monitor motion of a user. The sensor 102 may include any one of an accelerometer, a gyroscope, a pressure sensor, a microphone, a magnetometer, or an altimeter, and may further include any one of a luminance sensor, an optical sensor, or a proximity sensor. Any sensor that is configured to monitor motion of the user may be referred to as a motion sensor. Therefore, the cited example should not be interpreted as a limitation on this disclosure. For example, the sensor 103 is a biosensor, and is configured to monitor a biological signal of the user. The sensor 103 may include an electronic nose sensor (E-nose sensor), an EMG sensor (electromyography sensor, electromyogram sensor), an EEG sensor (electroencephalogram sensor, electroencephalogram sensor), an ECG sensor (electrocardiogram sensor, electrocardiogram sensor), or a fingerprint sensor. In addition, the sensor 102 and the sensor 103 may measure a physical quantity or sense an operating status of the electronic apparatus, and convert measured or sensed information into an electrical signal.

Optionally, the electronic device 100 may further include an input module 105. The input module 105 may receive a command or data from the user, and transfer the command or the data to the processor 101 or the memory 108 by using the bus 104. For example, the input module 105 may include a touchpad (touch panel), a key (key), or an ultrasonic input apparatus. The touchpad may identify touch input by using at least one of a capacitive manner, a pressure-sensing manner, an infrared manner, or an ultrasonic manner. The touchpad may further include a controller. In the capacitive manner, both direct touch and proximity may be identified. The touchpad may further include a tactile layer (tactile layer). In this case, the touchpad may provide the user with a tactile reaction. The key may include a keyboard or a touch key. The ultrasonic input apparatus may be an apparatus that senses, by using a pen that generates an ultrasonic signal, an ultrasonic wave in the electronic apparatus to acknowledge data, and may be configured to implement wireless identification.

Optionally, the electronic device 100 further includes a display module 106, and the display module 106 may display a graph, an image, or data to the user. For example, the display module 106 may include a panel. For example, the panel may be an LCD (liquid-crystal display, liquid crystal display), an LED (light emitting diode display, light emitting diode panel), or an AMOLED (active-matrix organic light-emitting diode, active-matrix organic light emitting diode). In addition, the panel may be constituted in a flexible (flexible), transparent (transparent), or wearable (wearable) form. The panel and the touchpad may also form one module. In addition, the display module 106 may further include a control circuit that is configured to control the panel. Optionally, the electronic device 100 further includes a communications module 107, so that the device 100 may communicate with one or more other electronic apparatuses or servers (not shown) by using any proper communications protocol. The communications module 107 may support a near field communication protocol such as Wi-Fi (wireless fidelity, Wireless Fidelity), Bluetooth (BT: Bluetooth), or near field communication (NFC: near field communication), the Internet (Internet), a local area network (LAN: local area network), a wide area network (WAN: wire area network), a telecommunication network (telecommunication network), a cellular network (cellular network), or a satellite network (satellite network). The communications module 107 may further include a circuit by using which the electronic device 100 can be coupled with another device (for example, a computer), and can communicate with the another device in a wired or wireless manner.

The bus 104 may be a circuit by using which constituent elements (for example, the processor 101, the memory 108, the sensor 102, the sensor 103, the input module 105, and the display module 106) included in the electronic device 100 are connected to each other, and communication is implemented between the constituent elements.

The processor 101 is configured to perform an instruction (for example, an instruction obtained from the input module 105), interrupt processing, timing, and another function. In addition, the processor 101 may include a graphics processing unit (graphic processing unit).

The memory 108 may store an instruction or data that is received by the processor 101 or another constituent element (for example, the input module 105, the display module 106, and the communications module 107) or that is generated by the processor 101 or another constituent element. In this case, the memory 108 may include an internal buffer and an external buffer.

In addition, the memory 108 may include a kernel, middleware, an application programming interface (API application programming interface). The kernel may control or manage a system resource (for example, the bus 104, the processor 101, or the memory 108) used to perform an action or a function implemented by another program module (for example, the middleware, the API, or an application). In addition, the kernel may provide an interface that is used to perform control or management by accessing, from the middleware, the API, or the application, an individual constituent element of the electronic device 100. The middleware may perform an intermediate function, so that the API or the application can communicate with the kernel to exchange data. In addition, the middleware may allocate a priority sequence of the system resource (for example, the bus 104, the processor 101, or the memory 108) of the electronic device 100 according to a working request received from at least one application, so as to execute load balancing (load balancing) for the working request. The API is an interface that is used to control, by using an application, a function provided by the kernel or the middleware, and may include at least one interface or function used for file control, window control, image processing, or word control.

FIG. 2 is a flowchart of a biological signal collection method according to an embodiment of the present invention. The method provided in this embodiment may be applied to the electronic device 100 shown in FIG. 1. The electronic device 100 may include but is not limited to a wearable device and other portable and non-portable computing devices, for example, a smart band, a smartwatch, a smartphone, a tablet computer, and a laptop computer. Referring to FIG. 2, the method includes the following steps.

Step S210: Obtain output data of at least one motion sensor.

In an optional implementation of this embodiment, motion status information of a user is obtained by using at least one motion sensor (for example, the sensor 102 in FIG. 1). In some embodiments, the motion sensor includes an accelerometer, a gyroscope, and a magnetometer. The accelerometer, the gyroscope, and the magnetometer may all measure data changes in three axial directions in three-dimensional space, and form a nine-axis posture detection sensor. In some implementations, the motion sensor 102 may be implemented as micro-electro-mechanical systems (MEMS).

In some embodiments, the output data of the motion sensor is original data. In some other embodiments, the output data of the motion sensor is processed data, for example, a motion direction and a motion speed of an electronic device that are calculated by using output data of a plurality of motion sensors.

In an optional implementation of this embodiment, the output data of the at least one motion sensor is obtained according to a preset time period. For example, the motion status information of the user is obtained once every one to three seconds.

In another optional implementation of this embodiment, only output data of the at least one motion sensor in a preset time period (for example, five seconds) is collected for use in subsequent information processing.

Step S220: Control collection duration of at least one biological signal of a user according to at least the output data of the at least one motion sensor.

In some embodiments, the electronic device includes at least one biological signal sensor (for example, the sensor 103 in FIG. 1) that can detect a biological signal of the user. The biological signal includes an electrocardiogram (ECG), an electroencephalogram (EEG), an electromyogram (EMG), electrical bioimpedance, temperature, blood glucose, blood oxygen, blood pressure, a photoplethysmogram (PPG), and the like.

The user actively starts a biological signal sensor with a particular function to start collecting biological signal information, or the electronic device controls a biosensor to periodically and automatically detect specified biological signal information. The information can be stored on the device, or be transmitted to a remote device by sharing the information with another device or by using network communication. For example, a user whose ECG and heart rate data are collected may need to touch several dry sensors (dry sensor) with both hands, or can use a capacitive (for example, non-contact) sensor that can collect the ECG and the heart rate data only by placing the sensor near a chest, or can use an oxymetric sensor (oxymetric sensor) to measure a heart rate at a fingertip.

Step S230: Collect the at least one biological signal of the user in the collection duration of the at least one biological signal.

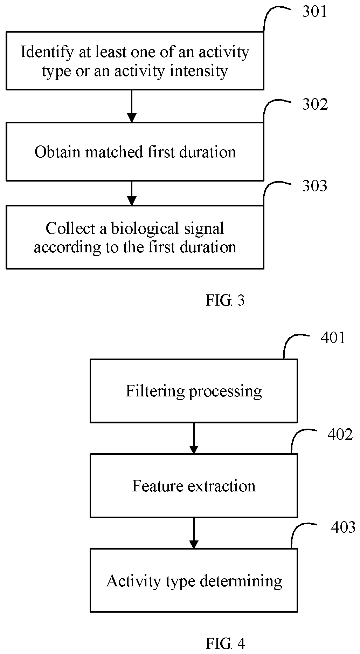

As shown in FIG. 3, in a possible implementation, step S220 may specifically include the following steps:

Step 301: Identify at least one of an activity type or an activity intensity of the user according to at least the output data of the at least one motion sensor.

Step 302: Obtain first duration that matches the at least one of the activity type or the activity intensity of the user.

Step 303: Collect the at least one biological signal of the user according to the first duration, and stop collecting the at least one biological signal when the first duration ends.

In an embodiment, a term "activity" may include various examples, such as running, walking, cycling, swimming, climbing, standing, sitting, and sleeping. Generally, any case that describes an action and/or movement of the user may be referred to as an "activity". Therefore, the cited example should not be interpreted as a limitation on this disclosure.

For different activity types and different activity intensities, a same biological signal differently changes and is differently affected by electromyogram noise and a motion artifact. Proper collection duration is selected according to a current activity type of the user or a current activity intensity of the user, so as to improve biological signal measurement precision.

In a possible implementation, in step 301, the activity type of the user may be determined in a method shown in FIG. 4. Referring to FIG. 4, the method includes the following steps.

Step 401: Perform filtering processing on the output data of the at least one motion sensor.

The output data of the motion sensor usually includes a lot of noise. Some data may be deleted from the obtained output data of the at least one motion sensor (for example, the sensor 102 in FIG. 1) by filtering processing or other processing. For example, data related to vibration is eliminated or reduced by filtering processing. Vibration is caused by a car, a train, or a ship. For example, when a person is in a car whose engineer is started and that does not move, the person is actually in a still state, and an inertial sensor still generates sensory data. For another example, a person has a slight body action during a conversation, and in this case, the inertial sensor also generates data related to vibration.

Step 402: Calculate an eigenvalue according to the output data of the at least one motion sensor.

For example, data of an inertial sensor such as a gyroscope or an accelerometer is collected, so as to calculate a group of eigenvalues according to each group of data. In a possible implementation, the eigenvalue includes a signal average value or a signal standard deviation calculated according to a signal source. The following briefly describes an eigenvalue calculation method by using the accelerometer as an example. The accelerometer detects acceleration values $(x, y, z)$ in three directions: an x-axis, a y-axis, and a z-axis. An eigenvalue in a collection period $T$ may be calculated according to the following formulas:

$$\text{Average value} = \sum_{i=1}^{n} X(i);$$

and

$$\text{Standard deviation} = \sqrt{\sum_{i=1}^{n}\left(X(i) - \overline{X}\right)^2 / (n-1)},$$

where

n is a quantity of collection points in the collection period T, i is a sequence number of a collection point, $X(i)$ is a signal value at a collection point, $1 \leq i \leq n$, and $\overline{X}$ is an average value.

Step 403: Determine the activity type of the user according to the eigenvalue calculated in step 402.

Optionally, the eigenvalue calculated in step 402 may be compared with a threshold, to determine the activity type of the user. For example, if a standard deviation of the accelerometer is greater than a threshold A, it is assumed that the user is running. Otherwise, if a standard deviation of the accelerometer is greater than a threshold B and less than a threshold A, it is assumed that the user is walking. Otherwise, it is assumed that the user is standing or sitting. In this way, the action types of the user may be distinguished from each other.

To improve accuracy of activity type identification, a modern machine learning method may be applied. Body motion sensor data and a correct activity type are used as input, and training is performed by using a machine learning model, to obtain a body motion identification model. A body activity type is identified by using these body motion features, to obtain an identified activity type corresponding to the sensor data, so as to improve a rate of activity type identification.

In a possible implementation, the activity type or the activity intensity of the user may also be identified according to other useful information. The other useful information includes navigation information (for example, location information and speed information), information from an input device (for example, audio data captured by a microphone, or image information captured by a camera), and a determined context (for example, a context is determined by using a screen touch operation or a key pressing operation of the user, a sound made by the user, or in another manner), to determine the activity type of the user). For example, a speed and a location of the user, and a path through which the user passes are determined according to output data of a GPS module, and the activity type of the user is determined more precisely according to the determined speed, location, and path.

In a possible implementation, for repeated motion that is performed at an interval of a preset period and that has rhythmicity, such as running and swimming, during running, a quantity of steps per unit time (a running frequency) may be determined, and during swimming, a quantity of strokes per unit time may be determined. The activity intensity is calculated according to a motion frequency, a movement amount of each movement in the repeated motion, and a weight of a detected person. Assuming that motion is running, a running speed obtained by multiplying a running frequency and a stride may be used as the activity intensity. For another example, assuming that motion is swimming, a product of an arm swinging frequency and an arm swinging amplitude may be used as the activity intensity. The activity intensity may also be represented by using a parameter such as a breathing frequency or an oxygen amount consumed per unit time. The cited example should not be interpreted as a limitation on this disclosure. A table of a mapping relationship between an activity intensity (or an activity type) of a user and biological signal collection duration may be established. When biological signal collection duration needs to be learned of, collection duration that best matches a current activity intensity (or a current activity type) is obtained by searching the table. A same activity type may correspond to a plurality of activity intensities. Collection duration for different activity intensities is different. Running is used as an example, and a mapping relationship table shown in Table 1 is established. The table is searched and collection duration that best matches a current activity state of the user is obtained.

Table 1

| Activity type | Activity intensity range (Running speed, unit: m/s) | Biological signal type | Collection duration |
|---|---|---|---|
| Running | (1, 2] | ECG | 15s |
| | (2, 3] | ECG | 20s |
| | (3, 4] | ECG | 25s |

In another possible implementation, the activity intensity of the user may be divided into levels. For example, two acceleration thresholds A1 and A2 are set, and A1<A2. If an acceleration value is less than the threshold A1, an activity intensity level of the user is low, and is marked as L; otherwise, if an acceleration value is greater than the threshold A1 and less than A2, an activity intensity level of the user is medium, and is marked as M; or if an acceleration value is greater than the threshold A2, a motion level of the user is high, and is marked as H. Noise is increasingly larger as the activity intensity level of the user is increasingly higher. Therefore, required biological signal collection duration is increasingly longer.

One activity type may correspond to a plurality of activity intensity levels. For example, assuming that motion is running, the user may jog, normally run, or sprint. For another example, assuming that motion is walking, the user may slowly walk, normally work, or quickly walk. In some embodiments, the activity type and the activity intensity level of the user may be simultaneously determined, and biological signal collection duration that best matches the current activity state of the user is obtained according to the determined activity type and activity intensity level. Running is used as an example, and a mapping relationship table shown in Table 2 is established. The table is searched and collection duration that best matches the current activity state of the user is obtained.

In a possible implementation, activity types of the user may be classified into a static type and a motion type. In a motion state, the user is easily affected by the electromyogram noise and the motion artifact. In this case, biological signal quality is worse than quality in a motionless state. A collection time longer than that in a static state may be set to improve biological signal measurement precision.

**Table 2**

| Activity type | | Activity intensity level | Biological signal type | Collection duration |
|---|---|---|---|---|
| Running | Jogging | L | ECG | 25s |
| | Normal running | M | ECG | 30s |
| | Sprinting | H | ECG | 55s |

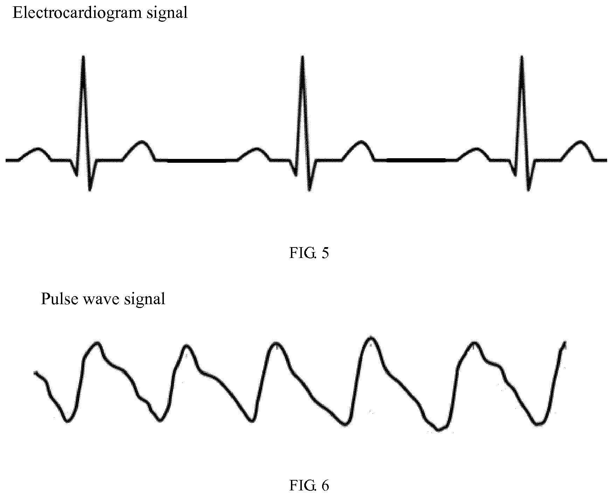

In some embodiments, a biological signal may be a periodic signal, and may include, for example, an electrocardiogram (ECG) signal, a pulse wave (PPG) signal, or another signal that has a period. For example, the biological signal may correspond to an ECG waveform shown in FIG. 5, or a PPG waveform shown in FIG. 6.

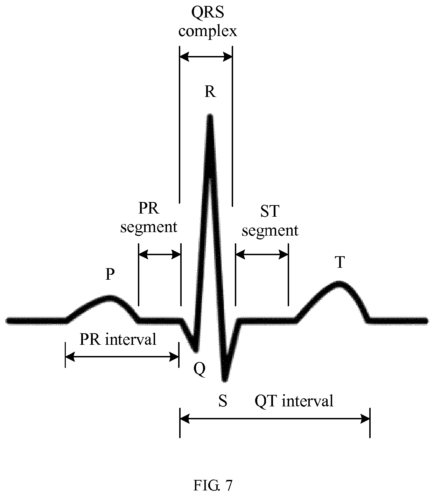

The ECG waveform may be a quasi-periodic signal that has a repeated pattern of a periodic PQRST waveform that includes a P wave, QRS waves, a T wave, and the like (shown in FIG. 7). There is also a U wave (a low voltage wavelet that is not shown) after the T wave. The P wave indicates an atrial depolarization process, a QRS complex indicates a ventricle depolarization process, and the T wave indicates a ventricle repolarization process. The QRS complex includes three closely connected waves. A first downwards deflected wave is referred to as a Q wave, a high and pointed upright standing wave following the Q wave is referred to as an R wave, and a downwards deflected wave after the R wave is referred to as an S wave. A time of a normal QRS complex is 0.06 to 0.10 second. A PR interval is a time from a start point of the P wave to a start point of the QRS complex. Generally, a PR interval of an adult is 0.12 to 0.20 second. The PR interval changes with a heart rate and an age, and a larger age usually indicates a longer PR interval. An S-T segment is a horizontal line from an endpoint of the QRS complex to a start point of the T wave. A QT interval is a period of time from the QRS complex to an end of the T wave.

A photoplethysmogram (PPG) is a wave formed by detecting a vascular capacity change in living tissue by using photoelectricity. Referring to FIG. 6, a pulse wave signal is also a deterministic signal close to a periodic signal.

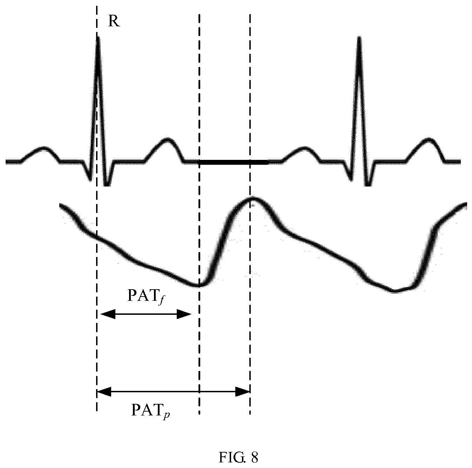

A pulse transit time (PTT) and a pulse arrival time (PAT) are usually used as parameters to determine blood pressure based on a PPG signal and an ECG signal. For example, based on a linear model of blood pressure and a pulse wave transit time (Pulse Transmit Time), that is PTT, the PTT may be calculated by using a delay time between a time at which the ECG is collected and a time at which the PPG is collected, where the ECG and the PPG are synchronously collected at two electrodes, so as to indirectly obtain a blood pressure value. An R-wave peak point of the ECG is extracted as a start point of the PTT, and a feature point of the PPG signal is used as an endpoint of the PTT. As shown in FIG. 8, the pulse wave arrival time (PAT) is a delay between the R-wave peak point of the ECG waveform and a corresponding feature point of the PPG waveform. For example, $PAT_f$ is a delay between the R-wave peak point of the ECG waveform and a trough of the PPG waveform, and $PAT_p$ is a delay between the R-wave peak point of the ECG waveform and a crest of the PPG waveform.

For a periodic biological signal, measurement precision usually can be ensured only when an enough quantity of complete waveforms are collected. For example, the pulse wave (PPG) signal is used to measure the heart rate. If collection duration is extremely short, a quantity of PPG waveforms may be insufficient, and consequently, a subsequent heart rate algorithm cannot be calculated or precision is extremely low. If collection duration is extremely long, a time of the user is easily wasted, because after a specified quantity of PPG waveforms are collected, optimal algorithm precision is achieved, and a subsequent PPG waveform has little effect on algorithm precision improvement, and may even

8

introduce noise that affects an algorithm result. In another aspect, because users have different heart rates, provided that a user having a high heart rate provides biological signals in a relatively short time, an enough quantity of PPG waveforms can be included, and an algorithm input requirement is met. However, relatively long collection duration is required if a user having a low heart rate needs to provide a same quantity of signals with a PPG waveform.

In some embodiments, for a periodic biological signal, to obtain an enough quantity of complete waveforms, a quantity of feature reference points of a collected biological signal may be detected. When the quantity of feature reference points reaches a specified quantity, biological signal collection is stopped, so as to ensure measurement precision. A feature reference point includes a crest point, a trough point, or another reference point.

For example, when the heart rate is measured, a feature reference point (a crest or a trough of a pulse wave) of the PPG signal is extracted to determine a quantity of collected complete pulse waves, pulse wave signal collection is stopped when a quantity of feature reference points is N (for example, N=15), and the heart rate is calculated according to a time required for collecting N complete pulse waveforms.

For another example, when the blood pressure is measured based on the PPG signal and the ECG signal, it is assumed that the pulse arrival time (PAT) is an input parameter, and $PAT_p$ is the delay between the R-wave peak point of the ECG waveform and the crest of the PPG waveform. A quantity of R-wave peak points of the ECG waveform and peak points of the PPG waveform is detected. When the quantity of R-wave peak points and peak points of the PPG waveform is M (for example, M=10), collection of the PPG signal and the ECG signal is stopped, and an average value of $PAT_p$ is obtained and used as an input parameter for calculating the blood pressure value.

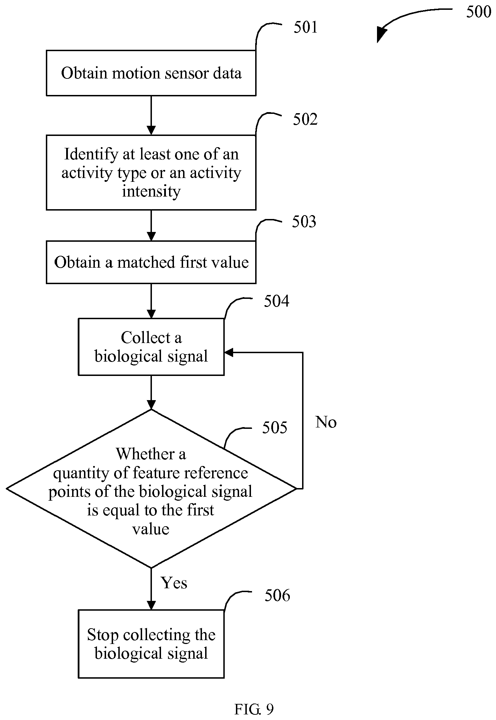

The following describes a procedure for collecting a periodic biological signal collection with reference to FIG. 9. Referring to FIG. 9, a method for obtaining motion sensor data in step 501 and a method for identifying an activity type and an activity intensity in step 502 are respectively the same as that in step S210 (in FIG. 2) and that in step 301 (in FIG. 3).

After a first value that matches at least one of the activity type or the activity intensity of the user (503), a biological signal is collected and a quantity of feature reference points of the at least one biological signal is calculated (504), and collection of the at least one biological signal is stopped (506) when the quantity of feature reference points is equal to the first value (505).

In some embodiments, a biological signal collection time period is divided into several relatively short time intervals. An average signal-to-noise ratio of biological signal data collected by each biological sensor at each time interval is obtained. When the average signal-to-noise ratio is greater than a specified determining threshold, the biological signal data at the time interval is stored as valid biological signal data.

For example, when a cortical electrocorticogram signal is measured, because a signal-to-noise ratio of the cortical electrocorticogram signal is relatively low, measurement of the cortical electrocorticogram signal is easily interfered with by electrooculogram noise, electromyogram noise, and other noise. If an average signal-to-noise ratio of cortical electrocorticogram signals at a time interval is less than a specified threshold, the time interval is used as an invalid collection time period; on the contrary, the time interval is a valid collection time period. The cortical electrocorticogram signal is collected, and until a sum of valid collection time periods is equal to specified duration, collection of the electrocorticogram signal is stopped.

For another example, when a heart rate is measured, a feature reference point (a crest or a trough of a pulse wave) of a PPG signal in each period is extracted. If an average signal-to-noise ratio of PPG signal data in a period is less than a specified determining threshold because of interference such as a motion noise, a feature reference point of a PPG signal in the period is used as an invalid feature reference point, and is not used as input for subsequent calculation of the heart rate. When a quantity of valid feature reference points is K (for example, K=10), pulse wave signal collection is stopped, and the heart rate is calculated according to K collected valid complete pulse waveforms. For example, a reciprocal of a time interval between every two valid feature reference points may be calculated to calculate an instant heart rate.

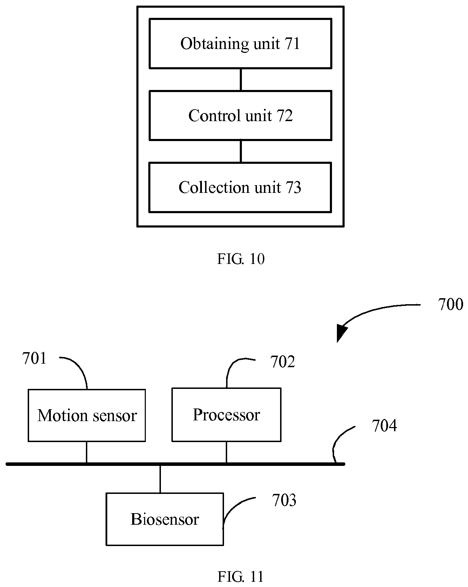

FIG. 10 is a schematic structural diagram of a biological signal collection apparatus according to an embodiment of the present invention. As shown in FIG. 10, the biological signal collection apparatus provided in this embodiment may implement all steps of a biological signal collection method that is provided in any embodiment of the present invention and that is applied to the biological signal collection apparatus. For a specific implementation process, details are not described herein. The biological signal collection apparatus provided in this embodiment specifically includes:

> an obtaining unit 71, configured to obtain output data of at least one motion sensor;
> a control unit 72, configured to control collection duration of at least one biological signal of a user according to at least the output data of the at least one motion sensor; and
> a collection unit 73, configured to collect the at least one biological signal of the user in the collection duration of the at least one biological signal.

In an optional implementation of this embodiment, the control unit 72 is specifically configured to:

identify at least one of an activity type or an activity intensity of the user according to at least the output data that is of the at least one motion sensor and that is obtained by the obtaining unit 71; and

obtain first duration that matches the at least one of the activity type or the activity intensity of the user, collect the at least one biological signal of the user according to the first duration, and stop collecting the at least one biological signal when the first duration ends.

In some embodiments, the at least one biological signal is periodic, and for a periodic biological signal, the control unit 72 is further configured to:

identify at least one of an activity type or an activity intensity of the user according to at least the output data that is of the at least one motion sensor and that is obtained by the obtaining unit 71; and

obtain a first value that matches the at least one of the activity type or the activity intensity of the user, detect a quantity of feature reference points of the at least one biological signal, and stop collecting the at least one biological signal when the quantity of feature reference points is equal to the first value.

Optionally, in this embodiment, the motion sensor includes any one of an accelerometer, a gyroscope, a pressure sensor, a microphone, a magnetometer, or an altimeter.

Optionally, in this embodiment, the activity type includes any one of running, walking, cycling, swimming, climbing, standing, sitting, or sleeping.

It may be understood that the biological signal collection apparatus herein is described by using a functional unit. The functional unit may be an application-specific integrated circuit (Application Specific Integrated Circuit, ASIC), an electronic circuit, or a processor. Particularly, the biological signal collection apparatus herein may be the electronic device 100 in FIG. 1. The obtaining unit 71 and the control unit 72 may be implemented by using the processor 101 and the memory 108, and the collection unit 73 may be implemented by using the biological signal sensor 103.

FIG. 11 shows a schematic structural diagram of another electronic device related to the foregoing embodiment. The following describes each constituent component of an electronic device 700 in detail with reference to FIG. 11. Referring to FIG. 11, the electronic device 700 includes:

at least one motion sensor 701, a memory 702, a processor 703, and at least one biosensor 704.

The at least one motion sensor 701 is configured to monitor motion of a user, and output data of the at least one motion sensor 701 is used as input of a subsequent algorithm for controlling biological signal collection duration.

The memory 702 is configured to store an instruction or data.

The processor 703 is configured to: obtain the output data of the at least one motion sensor 701, and control collection duration of at least one biological signal of the user according to at least the output data of the at least one motion sensor 701.

The processor 703 specifically performs processing processes, in FIG. 2 to FIG. 4 and FIG. 9, that are related to steps of obtaining output data of a motion sensor and controlling biological signal collection duration, and/or is configured to perform another process of the technology described in this application.

The at least one biosensor 704 is configured to collect the at least one biological signal of the user in the collection duration of the at least one biological signal.

It may be understood that FIG. 11 shows merely a simplified design of the electronic device. In actual application, the electronic device 700 may include the input module, the display module, and the communications module of the electronic device 100 in FIG. 1.

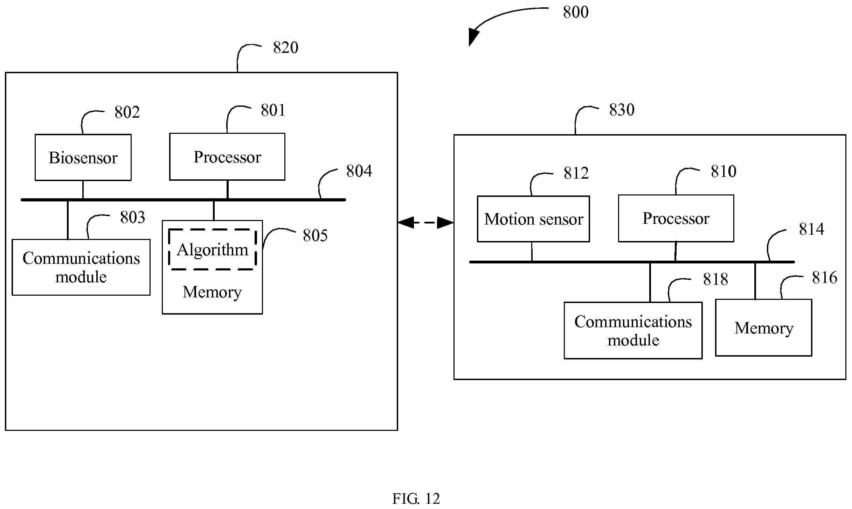

FIG. 12 is a schematic diagram of a system according to an embodiment of the present invention. Referring to FIG. 12, a system 800 includes a biological signal measurement device 820 and a motion signal measurement device 830. The two devices may be connected in a wireless or wired manner. The device 820 and the device 830 may include constituent elements of the electronic device shown in FIG. 1.

The system 800 includes at least one motion sensor 812, a memory 805, a processor 801, and at least one biosensor 802. The at least one motion sensor 812 is configured to monitor motion of a user.

The memory 805 is configured to store an instruction or data.

The processor 801 is coupled to the memory 805, and the processor 801 is configured to: obtain output data of the at least one motion sensor, and control collection duration of at least one biological signal of the user according to at least the output data of the at least one motion sensor.

The at least one biosensor 802 is configured to collect the at least one biological signal of the user in the collection duration of the at least one biological signal.

In a possible implementation, the at least one motion sensor 812 is coupled to the processor 801 by using a wireless interface.

In another possible implementation, the at least one motion sensor 812 is coupled to the processor 801 by using a wired interface.

In a possible implementation, the at least one biosensor 802 is coupled to the processor 801 by using a wireless interface.

In another possible implementation, the at least one biosensor 802 is coupled to the processor 801 by using a wired interface.

Optionally, the at least one motion sensor 812 and the processor 801 are disposed on a same device, or separately disposed on different devices.

Optionally, the at least one biosensor 802 and the processor 801 are disposed on a same device, or separately disposed on different devices.

In an embodiment, the device 830 may be attached to a leg of a user, and the device 820 may be attached to an arm of the user. The motion sensor 812 is configured to receive activity data, and the biosensor 802 is configured to detect a biological signal. The motion sensor 812 is correspondingly connected to a processor 810 that receives activity data from a motion sensor. The biosensor 802 is correspondingly connected to a processor 801 that receives a biological signal from a biosensor. Then the processor 810 provides data for a communications module 818 corresponding to the processor 810. The device 820 includes a communications module 803 that receives data from the communications module 818 and a memory 805 that includes a collection duration control algorithm.

The processor 801 further performs processing processes, in FIG. 2 to FIG. 4 and FIG. 9, that are related to steps of obtaining output data of a motion sensor and controlling biological signal collection duration, and/or is configured to perform another process of the technology described in this application.

It may be understood that FIG. 12 shows merely simplified designs of the biological signal measurement device 820 and the motion signal measurement device 830. In actual application, the device 820 and the device 830 each may further include any quantity of processors, memories, communications modules, sensors, and the like. The device 820 and the device 830 each may further include the input module and the display module in FIG. 1. All devices that can implement the present invention fall within the protection scope of the present invention.

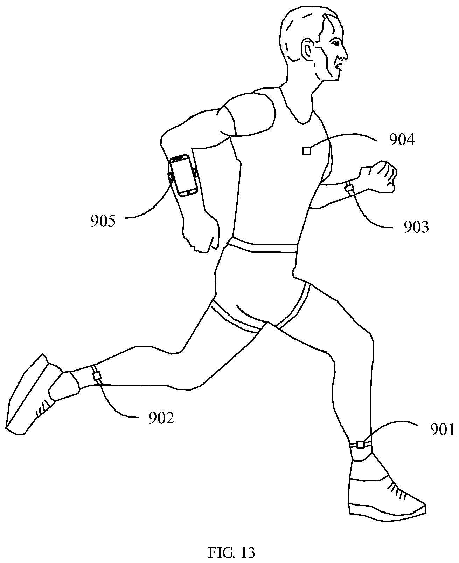

FIG. 13 is a schematic diagram of a specific application scenario of a system according to another embodiment of the present invention. A device 905 may be a mobile electronic device, for example a mobile phone, a tablet computer, or another similar electronic device mentioned above.

In an optional implementation, the device 905 may collect data of a motion sensor from a wearable device 901 and a wearable device 902. The device 905 calculates optimal measurement duration of each biological signal based on at least the collected data of the motion sensor and by using the biological signal collection duration control algorithm mentioned above, and provides the optimal measurement duration for a wearable device 903 and a wearable device 904 that measure a biological signal. For example, the wearable device 903 is configured to measure a pulse wave, and the wearable device 904 is configured to measure an electrocardiogram.

In another optional implementation, the wearable device 903 may directly collect data of a motion sensor from the wearable device 901 and the wearable device 902, and control biological signal measurement duration by using the biological signal collection duration control algorithm mentioned above.

Any two of the device 905, the wearable device 901, the wearable device 902, the wearable device 903, and the wearable device 904 may communicate with each other by using a wired or wireless communications protocol. For example, a protocol may be a short-range wireless communications protocol, for example, Bluetooth (Bluetooth), ZigBee, or ANT, or may be a long-range wired communications protocol, for example, a protocol in a computer communications field such as TCP/IP.

It may be understood that the wearable device in FIG. 13 may include a band, a watch, a ring, a button, and the like, and may be worn at any part of a human body. This is not limited in the present invention.

The processor of the electronic device and the system that are configured to execute the present invention may be a central processing unit (CPU), a general purpose processor, a digital signal processor (DSP), an application-specific integrated circuit (ASIC), a field programmable gate array (FPGA) or another programmable logical device, a transistor logical device, a hardware component, or any combination thereof. The processor may implement or execute various example logical blocks, modules, and circuits described with reference to content disclosed in the present invention. Alternatively, the processor may be a combination of processors implementing a computing function, for example, a combination of one or more microprocessors, or a combination of the DSP and a microprocessor.

Method or algorithm steps described in combination with the content disclosed in the present invention may be implemented by hardware, or may be implemented by a processor by executing a software instruction. The software instruction may be formed by a corresponding software module. The software module may be located in a RAM memory, a flash memory, a ROM memory, an EPROM memory, an EEPROM memory, a register, a hard disk, a removable magnetic disk, a CD-ROM, or a storage medium of any other form known in the art. For example, a storage medium is coupled to a processor, so that the processor can read information from the storage medium or write information into the storage medium. Certainly, the storage medium may be a component of the processor. The processor and the storage medium may be located in the ASIC. In addition, the ASIC may be located in user equipment. Certainly, the processor and the storage medium may exist in the user equipment as discrete components.

A person skilled in the art should be aware that in the foregoing one or more examples, functions described in the present invention may be implemented by hardware, software, firmware, or any combination thereof. A person skilled in the art should easily be aware that, in combination with the examples described in the embodiments disclosed in this specification, units and algorithm steps may be implemented by hardware or a combination of hardware and computer software. Whether a function is performed by hardware or hardware driven by computer software depends on particular applications and design constraints of the technical solutions. A person skilled in the art may use different methods to implement the described functions for each particular application, but it should not be considered that the implementation goes beyond the scope of the present invention. When the present invention is implemented by software, the foregoing functions may be stored in a computer-readable medium or transmitted as one or more instructions or code in the computer-readable medium. The computer-readable medium includes a computer storage medium and a communications medium, where the communications medium includes any medium that enables a computer program to be transmitted from one place to another. The storage medium may be any available medium accessible to a general-purpose or dedicated computer.

The objectives, technical solutions, and benefits of the present invention are further described in detail in the foregoing specific embodiments. It should be understood that the foregoing descriptions are specific embodiments of the present invention.

**Claims**

1. A software or hardware implemented biological signal collection method, comprising:

   obtaining (S210) output data of at least one motion sensor, wherein the at least one motion sensor comprises any one of a gyroscope, a pressure sensor, a microphone, a magnetometer, or an altimeter;
   controlling (S220) collection duration of at least one biological signal of a user according to at least the output data of the at least one motion sensor; and
   collecting (S230) the at least one biological signal of the user in the collection duration of the at least one biological signal,
   **characterized in that**
   the controlling (S220) collection duration of at least one biological signal of a user according to at least the output data of the at least one motion sensor comprises:

   identifying (301) an activity type and an activity intensity of the user according to at least the output data of the at least one motion sensor; and
   obtaining (302) a duration that matches the activity type and the activity intensity of the user, collecting (303) the at least one biological signal of the user according to the duration, and stopping collecting the at least one biological signal when the duration ends.

2. The method according to claim 1, wherein the at least one biological signal is periodic.

3. A software or hardware implemented biological signal collection method, comprising:

   obtaining (S210) output data of at least one motion sensor;
   controlling (S220) collection duration of at least one biological signal of a user according to at least the output data of the at least one motion sensor, wherein the at least one biological signal is periodic; and
   collecting (S230) the at least one biological signal of the user in the collection duration of the at least one biological signal;
   **characterized in that** the controlling (S220) collection duration of at least one biological signal of a user according to at least the output data of the at least one motion sensor comprises:

   identifying (502) an activity type and an activity intensity of the user according to at least the output data of the at least one motion sensor;
   obtaining (503) a value that matches the activity type and the activity intensity of the user, detecting (504) a quantity of feature reference points of the at least one biological signal, and stopping collecting (506) the at least one biological signal when the quantity of feature reference points is equal to the value.

4. The method according to claim 1 or 3, wherein the activity type comprises any one of running, walking, cycling, swimming, climbing, standing, sitting, or sleeping.

5. The method according to claim 3, wherein the motion sensor comprises any one of an accelerometer, a gyroscope, a pressure sensor, a microphone, a magnetometer, or an altimeter.

6. A biological signal collection system (800), comprising:

at least one motion sensor (812), a memory (805), a processor (801), and at least one biosensor (802), wherein the at least one motion sensor (812) is configured to monitor motion of a user and comprises any one of a gyroscope, a pressure sensor, a microphone, a magnetometer, or an altimeter;
the memory (805) is configured to store an instruction or data;
the processor (801) is coupled to the memory (805), and the processor (801) is configured to: obtain output data of the at least one motion sensor (812), and control collection duration of at least one biological signal of the user according to at least the output data of the at least one motion sensor (812); and
the at least one biosensor (802) is configured to collect the at least one biological signal of the user in the collection duration of the at least one biological signal,
**characterized in that** the controlling collection duration of at least one biological signal of the user according to at least the output data of the at least one motion sensor comprises:

identifying an activity type and an activity intensity of the user according to at least the output data of the at least one motion sensor; and
obtaining a duration that matches the activity type and the activity intensity of the user, collecting the at least one biological signal of the user according to the duration, and stopping collecting the at least one biological signal when the duration ends.

7. The system (800) according to claim 6, wherein the at least one biological signal is periodic.

8. A biological signal collection system (800), comprising:

at least one motion sensor (812), a memory (805), a processor (801), and at least one biosensor (802), wherein the at least one motion sensor (812) is configured to monitor motion of a user;
the memory (805) is configured to store an instruction or data;
the processor (801) is coupled to the memory (805), and the processor (801) is configured to: obtain output data of the at least one motion sensor (812), and control collection duration of at least one biological signal of the user according to at least the output data of the at least one motion sensor (812), wherein the at least one biological signal is periodic; and
the at least one biosensor (802) is configured to collect the at least one biological signal of the user in the collection duration of the at least one biological signal,
**characterized in that** the controlling collection duration of at least one biological signal of the user according to at least the output data of the at least one motion sensor comprises:
identifying an activity type and an activity intensity of the user according to at least the output data of the at least one motion sensor;
obtaining a value that matches the activity type and the activity intensity of the user,
detecting a quantity of feature reference points of the at least one biological signal, and stopping collecting the at least one biological signal when the quantity of feature reference points is equal to the value.

9. The system according to any one of claims 6 to 8, wherein the at least one motion sensor (812) is coupled to the processor (801) by using a wireless interface.

10. The system according to any one of claims 6 to 8, wherein the system is implemented in an electronic device.

**Patentansprüche**

1. Software- oder Hardware-implementiertes Verfahren zum Erfassen eines biologischen Signals, das Folgendes umfasst:

Erhalten (S210) von Ausgabedaten wenigstens eines Bewegungssensors, wobei der wenigstens eine Bewegungssensor ein beliebiges aus einem Gyroskop, einem Drucksensor, einem Mikrofon, einem Magnetometer oder einem Höhenmesser umfasst;

Steuern (S220) der Erfassungsdauer wenigstens eines biologischen Signals eines Anwenders wenigstens gemäß den Ausgabedaten des wenigstens einen Bewegungssensors; und

Erfassen (S230) des wenigstens einen biologischen Signals des Anwenders in der Erfassungsdauer des wenigstens einen biologischen Signals,

**dadurch gekennzeichnet, dass**

das Steuern (S220) der Erfassungsdauer wenigstens eines biologischen Signals eines Anwenders wenigstens gemäß den Ausgabedaten des wenigstens einen Bewegungssensors Folgendes umfasst:

Identifizieren (301) eines Aktivitätstyps und einer Aktivitätsintensität des Anwenders wenigstens gemäß den Ausgabedaten des wenigstens einen Bewegungssensors; und

Erhalten (302) einer Dauer, die mit dem Aktivitätstyp und der Aktivitätsintensität des Anwenders übereinstimmt,

Erfassen (303) des wenigstens einen biologischen Signals des Anwenders gemäß der Dauer und Beenden des Erfassens des wenigstens einen biologischen Signals, wenn die Dauer endet.

2. Verfahren nach Anspruch 1, wobei das wenigstens eine biologische Signal periodisch ist.

3. Software- oder Hardware-implementiertes Verfahren zum Erfassen eines biologischen Signals, das Folgendes umfasst:

Erhalten (S210) von Ausgabedaten wenigstens eines Bewegungssensors;

Steuern (S220) der Erfassungsdauer wenigstens eines biologischen Signals eines Anwenders wenigstens gemäß den Ausgabedaten des wenigstens einen Bewegungssensors, wobei das wenigstens eine biologische Signal periodisch ist; und

Erfassen (S230) des wenigstens einen biologischen Signals des Anwenders in der Erfassungsdauer des wenigstens einen biologischen Signals;

**dadurch gekennzeichnet, dass**

das Steuern (S220) der Erfassungsdauer wenigstens eines biologischen Signals eines Anwenders wenigstens gemäß den Ausgabedaten des wenigstens einen Bewegungssensors Folgendes umfasst:

Identifizieren (502) eines Aktivitätstyps und einer Aktivitätsintensität des Anwenders wenigstens gemäß den Ausgabedaten des wenigstens einen Bewegungssensors;

Erhalten (503) eines Wertes, der mit dem Aktivitätstyp und der Aktivitätsintensität des Anwenders übereinstimmt,

Detektieren (504) einer Anzahl von Merkmalsbezugspunkten des wenigstens einen biologischen Signals, und

Beenden des Erfassens (506) des wenigstens einen biologischen Signals, wenn die Anzahl der Merkmalsbezugspunkte gleich dem Wert ist.

4. Verfahren nach Anspruch 1 oder 3, wobei der Aktivitätstyp eines aus Laufen, Gehen, Radfahren, Schwimmen, Klettern, Stehen, Sitzen oder Schlafen umfasst.

5. Verfahren nach Anspruch 3, wobei der Bewegungssensor ein beliebiges aus einem Beschleunigungsmesser, einem Gyroskop, einem Drucksensor, einem Mikrofon, einem Magnetometer oder einem Höhenmesser umfasst.

6. System (800) zum Erfassen eines biologischen Signals, das Folgendes umfasst:

wenigstens einen Bewegungssensor (812), einen Speicher (805), einen Prozessor (801) und wenigstens einen Biosensor (802), wobei

der wenigstens eine Bewegungssensor (812) konfiguriert ist, die Bewegung eines Anwenders zu überwachen, und ein beliebiges aus einem Gyroskop, einem Drucksensor, einem Mikrofon, einem Magnetometer oder einem Höhenmesser umfasst;

der Speicher (805) konfiguriert ist, eine Anweisung oder Daten zu speichern;

der Prozessor (801) mit dem Speicher (805) gekoppelt ist und der Prozessor (801) konfiguriert ist zum: Erhalten von Ausgabedaten des wenigstens einen Bewegungssensors (812) und Steuern der Erfassungsdauer wenigstens eines biologischen Signals des Anwenders wenigstens gemäß den Ausgabedaten des wenigstens einen Bewegungssensors (812); und

der wenigstens eine Biosensor (802) konfiguriert ist, das wenigstens eine biologische Signal des Anwenders in der Erfassungsdauer des wenigstens einen biologischen Signals zu erfassen,
**dadurch gekennzeichnet, dass**
das Steuern der Erfassungsdauer wenigstens eines biologischen Signals des Anwenders wenigstens gemäß den Ausgabedaten des wenigstens einen Bewegungssensors Folgendes umfasst:

Identifizieren eines Aktivitätstyps und einer Aktivitätsintensität des Anwenders wenigstens gemäß den Ausgabedaten des wenigstens einen Bewegungssensors; und
Erhalten einer Dauer, die mit dem Aktivitätstyp und der Aktivitätsintensität des Anwenders übereinstimmt, Erfassen des wenigstens einen biologischen Signals des Anwenders gemäß der Dauer und Beenden des Erfassens des wenigstens einen biologischen Signals, wenn die Dauer endet.

7. System (800) nach Anspruch 6, wobei das wenigstens eine biologische Signal periodisch ist.

8. System (800) zum Erfassen eines biologischen Signals, das Folgendes umfasst:

wenigstens einen Bewegungssensor (812), einen Speicher (805), einen Prozessor (801) und wenigstens einen Biosensor (802), wobei
der wenigstens eine Bewegungssensor (812) konfiguriert ist, die Bewegung eines Anwenders zu überwachen;
der Speicher (805) konfiguriert ist, eine Anweisung oder Daten zu speichern;
der Prozessor (801) mit dem Speicher (805) gekoppelt ist und der Prozessor (801) konfiguriert ist zum: Erhalten von Ausgabedaten des wenigstens einen Bewegungssensors (812) und Steuern der Erfassungsdauer wenigstens eines biologischen Signals des Anwenders wenigstens gemäß den Ausgabedaten des wenigstens einen Bewegungssensors (812), wobei das wenigstens eine biologische Signal periodisch ist; und
der wenigstens eine Biosensor (802) konfiguriert ist, das wenigstens eine biologische Signal des Anwenders in der Erfassungsdauer des wenigstens einen biologischen Signals zu erfassen,
**dadurch gekennzeichnet, dass**
das Steuern der Erfassungsdauer wenigstens eines biologischen Signals des Anwenders wenigstens gemäß den Ausgabedaten des wenigstens einen Bewegungssensors Folgendes umfasst:

Identifizieren eines Aktivitätstyps und einer Aktivitätsintensität des Anwenders wenigstens gemäß den Ausgabedaten des wenigstens einen Bewegungssensors;
Erhalten eines Wertes, der mit dem Aktivitätstyp und der Aktivitätsintensität des Benutzers übereinstimmt, Detektieren einer Anzahl von Merkmalsbezugspunkten des wenigstens einen biologischen Signals und Beenden des Erfassens des wenigstens einen biologischen Signals, wenn die Anzahl der Merkmalsbezugspunkte gleich dem Wert ist.

9. System nach einem der Ansprüche 6 bis 8, wobei der wenigstens eine Bewegungssensor (812) unter Verwendung einer Drahtlosschnittstelle mit dem Prozessor (801) gekoppelt ist.

10. System nach einem der Ansprüche 6 bis 8, wobei das System in einer elektronischen Vorrichtung implementiert ist.

**Revendications**

1. Procédé de collecte de signaux biologiques à mise en oeuvre logicielle ou matérielle, comprenant :

l'obtention (S210) de données de sortie d'au moins un capteur de mouvement, l'au moins un capteur de mouvement comprenant un élément quelconque parmi un gyroscope, un capteur de pression, un microphone, un magnétomètre ou un altimètre ;
la commande (S220) d'une durée de collecte d'au moins un signal biologique d'un utilisateur selon au moins les données de sortie de l'au moins un capteur de mouvement ; et
la collecte (S230) de l'au moins un signal biologique de l'utilisateur dans la durée de collecte de l'au moins un signal biologique,
**caractérisé en ce que**
la commande (S220) d'une durée de collecte d'au moins un signal biologique d'un utilisateur selon au moins les données de sortie de l'au moins un capteur de mouvement comprend :

l'identification (301) d'un type d'activité et d'une intensité d'activité de l'utilisateur selon au moins les données de sortie de l'au moins un capteur de mouvement ; et

l'obtention (302) d'une durée qui correspond au type d'activité et à l'intensité d'activité de l'utilisateur, la collecte (303) de l'au moins un signal biologique de l'utilisateur selon la durée, et l'arrêt de la collecte de l'au moins un signal biologique lorsque la durée prend fin.

2. Procédé selon la revendication 1, dans lequel l'au moins un signal biologique est périodique.

3. Procédé de collecte de signaux biologiques à mise en oeuvre logicielle ou matérielle, comprenant :

l'obtention (S210) de données de sortie d'au moins un capteur de mouvement ;
la commande (S220) d'une durée de collecte d'au moins un signal biologique d'un utilisateur selon au moins les données de sortie de l'au moins un capteur de mouvement, l'au moins un signal biologique étant périodique ; et
la collecte (S230) de l'au moins un signal biologique de l'utilisateur dans la durée de collecte de l'au moins un signal biologique ;
**caractérisé en ce que**
la commande (S220) d'une durée de collecte d'au moins un signal biologique d'un utilisateur selon au moins les données de sortie de l'au moins un capteur de mouvement comprend :

l'identification (502) d'un type d'activité et d'une intensité d'activité de l'utilisateur selon au moins les données de sortie de l'au moins un capteur de mouvement ;
l'obtention (503) d'une valeur qui correspond au type d'activité et à l'intensité d'activité de l'utilisateur,
la détection (504) d'une quantité de points de référence de caractéristique de l'au moins un signal biologique, et
l'arrêt de la collecte (506) de l'au moins un signal biologique lorsque la quantité de points de référence de caractéristique est égale à la valeur.

4. Procédé selon la revendication 1 ou 3, dans lequel le type d'activité comprend un élément quelconque parmi la course, la marche, le cyclisme, la natation, l'escalade, la position debout, la position assise ou le sommeil.

5. Procédé selon la revendication 3, dans lequel le capteur de mouvement comprend un élément quelconque parmi un accéléromètre, un gyroscope, un capteur de pression, un microphone, un magnétomètre ou un altimètre.

6. Système (800) de collecte de signaux biologiques, comprenant :

au moins un capteur de mouvement (812), une mémoire (805), un processeur (801) et au moins un biocapteur (802),
l'au moins un capteur de mouvement (812) étant configuré pour surveiller le mouvement d'un utilisateur et comprenant un élément quelconque parmi un gyroscope, un capteur de pression, un microphone, un magnétomètre ou un altimètre ;
la mémoire (805) étant configurée pour stocker une instruction ou des données ;
le processeur (801) étant couplé à la mémoire (805), et le processeur (801) étant configuré pour : obtenir des données de sortie de l'au moins un capteur de mouvement (812) et commander une durée de collecte d'au moins un signal biologique de l'utilisateur selon au moins les données de sortie de l'au moins un capteur de mouvement (812) ; et
l'au moins un biocapteur (802) étant configuré pour collecter l'au moins un signal biologique de l'utilisateur dans la durée de collecte de l'au moins un signal biologique, **caractérisé en ce que**
la commande d'une durée de collecte d'au moins un signal biologique de l'utilisateur selon au moins les données de sortie de l'au moins un capteur de mouvement comprend :

l'identification d'un type d'activité et d'une intensité d'activité de l'utilisateur selon au moins les données de sortie de l'au moins un capteur de mouvement ; et
l'obtention d'une durée qui correspond au type d'activité et à l'intensité d'activité de l'utilisateur, la collecte de l'au moins un signal biologique de l'utilisateur selon la durée, et l'arrêt de la collecte de l'au moins un signal biologique lorsque la durée prend fin.

7. Système (800) selon la revendication 6, dans lequel l'au moins un signal biologique est périodique.

**8.** Système (800) de collecte de signaux biologiques, comprenant :

au moins un capteur de mouvement (812), une mémoire (805), un processeur (801) et au moins un biocapteur (802),

l'au moins un capteur de mouvement (812) étant configuré pour surveiller le mouvement d'un utilisateur ;

la mémoire (805) étant configurée pour stocker une instruction ou des données ;

le processeur (801) étant couplé à la mémoire (805), et le processeur (801) étant configuré pour : obtenir des données de sortie de l'au moins un capteur de mouvement (812) et commander une durée de collecte d'au moins un signal biologique de l'utilisateur selon au moins les données de sortie de l'au moins un capteur de mouvement (812), l'au moins un signal biologique étant périodique ; et

l'au moins un biocapteur (802) étant configuré pour collecter l'au moins un signal biologique de l'utilisateur dans la durée de collecte de l'au moins un signal biologique, **caractérisé en ce que**

la commande d'une durée de collecte d'au moins un signal biologique de l'utilisateur selon au moins les données de sortie de l'au moins un capteur de mouvement comprend :

l'identification d'un type d'activité et d'une intensité d'activité de l'utilisateur selon au moins les données de sortie de l'au moins un capteur de mouvement ;

l'obtention d'une valeur qui correspond au type d'activité et à l'intensité d'activité de l'utilisateur,

la détection d'une quantité de points de référence de caractéristique de l'au moins un signal biologique, et

l'arrêt de la collecte de l'au moins un signal biologique lorsque la quantité de points de référence de caractéristique est égale à la valeur.

**9.** Système selon l'une quelconque des revendications 6 à 8, dans lequel l'au moins un capteur de mouvement (812) est couplé au processeur (801) au moyen d'une interface sans fil.

**10.** Système selon l'une quelconque des revendications 6 à 8, le système étant mis en oeuvre dans un dispositif électronique.

100

| 102 | 103 | 101 |
|-----|-----|-----|
| Sensor | Sensor | Processor |

104

| 105 | 106 | 107 | 108 |
|-----|-----|-----|-----|
| Input module | Display module | Communications module | Memory |

FIG. 1

| | |
|---|---|
| Obtain output data of at least one motion sensor | S210 |
| Control collection duration of at least one biological signal of a user according to at least the output data of the at least one motion sensor | S220 |
| Collect the at least one biological signal of the user in the collection duration of the at least one biological signal | S230 |

FIG. 2

301

Identify at least one of an activity
type or an activity intensity

302

Obtain matched first duration

303

Collect a biological signal
according to the first duration

FIG. 3

401

Filtering processing

402

Feature extraction

403

Activity type determining

FIG. 4

Electrocardiogram signal

FIG. 5

Pulse wave signal

FIG. 6

FIG. 7

FIG. 8

500

501

Obtain motion sensor data

502

Identify at least one of an
activity type or an activity
intensity

503

Obtain a matched first value

504

Collect a
biological signal

505

Whether a
quantity of feature reference
points of the biological signal
is equal to the first
value

No

Yes

506

Stop collecting the
biological signal

FIG. 9

| Obtaining unit 71 |
| Control unit 72 |
| Collection unit 73 |

FIG. 10

700

701

| Motion sensor | Processor | 702

704

703

| Biosensor |

FIG. 11

FIG. 12

FIG. 13

**EP 3 375 357 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015058923 A1
- WO 2015131065 A1
- WO 2016010652 A1